Europäisches Patentamt

(19) European Patent Office

Office européen des brevets

(11) Publication number: **0 026 679**
**B1**

(12) **EUROPEAN PATENT SPECIFICATION**

(45) Date of publication of patent specification: **29.08.84**

(51) Int. Cl.³: **A 61 B 5/14**

(21) Application number: **80303482.6**

(22) Date of filing: **02.10.80**

(54) **Improvements relating to blood sampling apparatus.**

(30) Priority: **02.10.79 IE 1877/79**
**14.11.79 IE 2187/79**
**21.12.79 IE 2510/79**
**25.02.80 IE 373/80**
**25.04.80 IE 855/80**
**20.08.80 IE 1758/80**
**29.08.80 IE 1837/80**

(43) Date of publication of application:
**08.04.81 Bulletin 81/14**

(45) Publication of the grant of the patent:
**29.08.84 Bulletin 84/35**

(84) Designated Contracting States:
**AT CH DE FR GB IT LI LU NL SE**

(56) References cited:
**FR-A-1 337 891**
**FR-A-1 416 487**
**FR-A-1 511 044**
**GB-A-1 059 257**

(73) Proprietor: **Stafford, Rodney Arthur**
**Cloon House**
**Glencree Co. Wicklow (IE)**
(73) Proprietor: **Kilroy, Michael Maxwell**
**Castlecor**
**Oldcastle Meath (IE)**

(72) Inventor: **Bennett, Michael Benedict**
**147 Blackberry Rise**
**Portmarnock County Dublin (IE)**
Inventor: **Stafford, Rodney Arthur**
**Cloon House Glencree**
**Co. Wicklow (IE)**
Inventor: **Kilroy, Michael Maxwell**
**Castlecor**
**Oldcastle County Meath (IE)**

(74) Representative: **Jennings, Roy Alfred**
**GILL JENNINGS & EVERY 53-64 Chancery Lane**
**London WC2A 1HN (GB)**

Courier Press, Leamington Spa, England.

## Description

This invention relates to blood sampling apparatus for use in obtaining blood samples from human beings or animals, particularly cattle.

Blood sampling apparatus in current use for vacuophlebotomy comprises 3 elements: a glass tube, closed by a butyl rubber stopper, the tube being evacuated prior to use; a needle holder in the form of a cylindrical shell closed at one end and having an external flange at the other end, the shell having a sufficiently large internal diameter to fit loosely over the stoppered end of the tube; and a tubular hypodermic needle with a point at each end (one pointed end being protected by a sheath), the needle being adapted to be passed partially through, and removably secured in, the closed end portion of the needle holder.

Such blood sampling apparatus is supplied, for example, to veterinary surgeons, in a pack containing a substantial number of tubes (e.g. 50 tubes), an equal number of needles, and a small number of needle holders (e.g. 2 holders). When a sample is to be taken, the veterinary surgeon or his assistant takes a needle from the pack, screws it into a needle holder, and takes an evacuated tube from the pack, and places the holder loosely on the stoppered end of the tube. While holding the tube and holder in his hand, the surgeon removes the sheath from the needle, locates a vein (particularly the jugular vein) in the animal, inserts the outer needle point into the vein, and by gripping the flange on the holder he presses the tube into the holder so that the inner needle point penetrates the stopper. The evacuated interior of the tube is then connected via the holder and needle with the vein, and blood is sucked from the vein into the tube. The surgeon then withdraws the tube from the holder and the rubber stopper seals back across the hole left by the needle. The tube containing the sample is replaced in the pack, to be sent for analysis. The neede should be unscrewed from the holder and disposed of safely. The holder is used again as further samples are taken.

This procedure using the known apparatus has certain disadvantages and indeed dangers:

1) The surgeon or his assistant is obliged to assemble the three components before each sample is taken. This can be troublesome when a large number of samples are to be taken particularly among restless animals.

2) The tube is not retained in the holder after the components have been assembled and there is nothing to prevent the tube falling out of the holder. It is customary therefore to insert the tip of the inner end of the needle into the rubber stopper, merely to hold the assembly together until it is required for use. Under the pressure of sample-taking in the field, there is a risk that the needle will be inserted too far into the stopper and will release the vacuum prematurely.

3) Used needles are sometimes dropped or even thrown away on the ground and there is a danger of needles (or fragments of needles) piercing the feet of the animals.

4) In cases of urgency, the surgeon or his assistant may (inadvertently or otherwise) omit the replacement of the needle in the holder and may use the same needle for taking two or more samples, even from different animals. This procedure involves a high risk of contamination and the spread of disease, particularly leucosis. Although the disadvantages above have been described with reference to veterinary use, it will be apparent that comparable problems can also arise with apparatus for taking blood samples from human beings.

It is an object of the present invention to overcome the above-mentioned disadvantages. Although the general nature of this problem has been appreciated for a number of years (see U.S. Patent No. 3,908,638), the standard blood sampling apparatus has continued in use.

There have been various proposals for unitary blood sampling assemblies e.g. in British Patents Nos. 1,059,257 and 1,036,000, U.S. Patent No. 3,123,073 and French Patent No. 1,278,387. However, so far as the present inventors are aware, none of these assemblies are available on the market. Accordingly, the present invention provides blood sampling apparatus supplied as an assembled unit comprising an evacuated tube with a stopper, and a needle holder in which a hollow needle or cannula is secured, said needle holder having a skirt which surrounds the stoppered end of the tube, characterised in that the stopper has a head with an external diameter not greater than the external diameter of the tube, and the interior of the skirt of the needle holder is adapted to embrace the exterior of the tube, preferably sufficiently tightly to prevent the tube falling out under the force of gravity, but nevertheless to allow the tube to slide inside the holder under manual pressure. The needle holder is also preferably of such a shape that air can escape from the interior of the holder around the tube. For example, the interior of the skirt may have longitudinal ribs which engage the tube while the spaces between said ribs provide air gaps between the tube and the holder. Alternatively the interior of the skirt may have longitudinal grooves. The skirt is a sliding friction fit on the tube. The use of ribs is advantageous because it reduces the surface area of the skirt in contact with the tube so that the extent of frictional engagement can be controlled.

It is advantageous to have the sliding contact made between the holder and the tube, rather than between the holder and the stopper (as in British Patent No. 1,059,257) because of the greater length of the contact area on the tube (which helps to keep the components in axial alignment) and because a frictional engagement between the stopper and the holder may lead to a risk of the stopper being withdrawn prematurely from the tube when the holder is being removed after a sample has been taken.

Preferably the holder has no external flange at its open end, although it may have an external bead. Preferably the closed end of the holder is formed as a finger grip. Most suitably, the holder is of reduced length (as compared to the standard needle holders in current use) and the length of the combination of holder and tube, prior to the stopper being punctured by the cannula, is such that a surgeon can hold the assembly in one hand with fingers gripping the closed end of the holder and with the closed end of the tube in the palm of his hand.

Usually the needle is a metal cannula having two pointed ends. However in one embodiment the needle has one pointed end outside the holder and the plastics holder itself has an internal axial cannula portion with a pointed end inside the skirt of the holder, to pierce the stopper, the bore of said cannula portion communicating with the bore of the needle.

According to another feature, the present invention provides blood sampling apparatus in a pack comprising a plurality of evacuated tubes each with a holder and needle in situ thereon. Preferably each tube stands in a cylindrical hole in a suitable packing material such as plastics foam. The packing material may suitably be provided with a ledge around each tube-receiving hole, the ledge being adapted to receive the lower end of the needle holder.

It is a valuable feature of the present invention that each tube is equipped with a needle holder and needle in situ thereon, so that there is no temptation for the person taking the sample to use the same needle twice. Each needle may be permanently secured to its holder, e.g. by adhesives or the like, by welding such as sonic welding or heat welding, or by being integrated into the holder during the moulding thereof. The needle may be directly secured in the holder or it may have a collar or hub thereon which is secured in the holder. The needle or the collar may be a snap fit or friction fit or push fit or other tight fit therein. If appropriate, the fitting of the needle into the holder may be tool-assisted.

Several embodiments of the invention will now be described with reference to the accompanying drawings, in which:

Figure 1 is a longitudinal cross section of one form of blood sampling apparatus in accordance with the invention, in situ in a portion of packing material, the cross section being taken on the line B—B in Figure 2;

Figure 2 is a transverse cross section of the needle holder skirt on the line A—A in Figure 1;

Figures 3 and 4 are sketch drawings illustrating the manner of use of the apparatus;

Figure 5 is an elevation of a needle holder in a second embodiment of the invention;

Figure 6 is a longitudinal cross section of a second form of blood sampling apparatus in accordance with the invention on the line E—E in Figure 7;

Figure 7 is a transverse cross section of the needle holder on the line F—F in Figure 6;

Figure 8 is a longitudinal cross section of a third form of blood sampling apparatus in accordance with the invention on the line G—G in Figure 9;

Figure 9 is a transverse cross section of the needle holder on the line H—H in Figure 8;

Figure 10 is a longitudinal cross section of a fourth form of blood sampling apparatus;

Figure 11 is a transverse cross section of the needle holder on the line D—D in Figure 10.

Figure 12 is a longitudinal cross section of a fifth embodiment of a needle holder according to the invention;

Figure 13 is a longitudinal cross section of a sixth embodiment of a needle holder according to the invention.

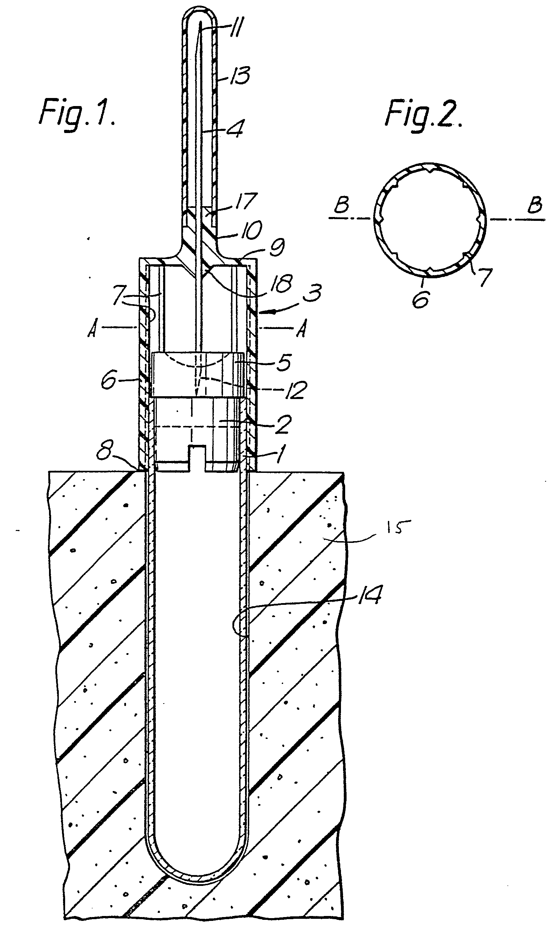

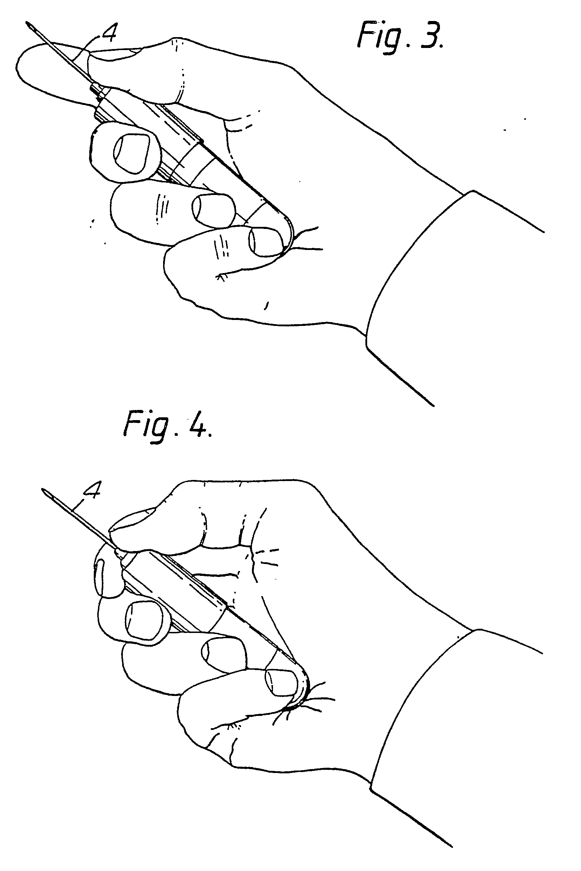

As shown in Figures 1 and 2, a blood sampling unit comprises an evacuated tube 1 having a stopper 2 and a needle holder 3 having a sterilized hollow needle 4 permanently secured therein.

The tube 1 is suitably a conventional glass tube, although a tube of plastics material may also be used. The stopper 2 of elastomeric material (suitably butyl rubber) is similar to a conventional stopper but the head portion 5 of the stopper has an external diameter marginally less than that of the tube 1 (although the external diameter of the head portion is greater than the internal diameter of the tube). If the head portion is tapered upwardly the widest part of the head seated on the tube may be of the same diameter as the tube.

The holder 3 is suitably moulded from a plastics material such as polypropylene, high density polyethylene or crystal polystyrene. The skirt 6 of the holder is a cylindrical shell, the internal surface of which is provided with eight longitudinal ribs 7. The lower end 8 of the skirt 6 does not have a rim or flange.

The holder is shorter and slimmer and has less wall thickness than the standard needle holders used hitherto, and it can be moulded easily and at low cost.

The closed portion of the holder is formed as a cap 9 of solid plastics material with an axial boss 10 extending upwardly therefrom. The boss has an upper portion 17 which is of reduced external diameter. A sleeve portion 18 extends downwardly from the cap. The needle 4, having points 11, 12 at the two ends, extends through the cap 9 so that the inner end 12 is inside the holder and just embedded in the stopper 2. The needle is permanently secured in the cap 9; for example the needle may be integrated into the holder during the moulding thereof or may be held therein by adhesive, welding or the like.

The outer end 11 of the needle is protected by a sheath 13 which fits on to the upper portion 17 of the boss 10 and is sealed thereto during manufacture.

In Figure 1, the tube 1 is shown in situ in a cylindrical hole 14 in packing material 15 which is suitably polystyrene foam. The lip 8 of the holder rests on the top surface of the packing material 15, so that the holder can not be pushed down on to the tube while the unit is stored in the pack.

The exterior surface of the holder may be roughened e.g. by spark erosion to assist gripping. Alternatively it may have a non-slip surface provided by grooves and/or ridges lying, for example, in a plane perpendicular to the axis of the holder, or lying helically or axially.

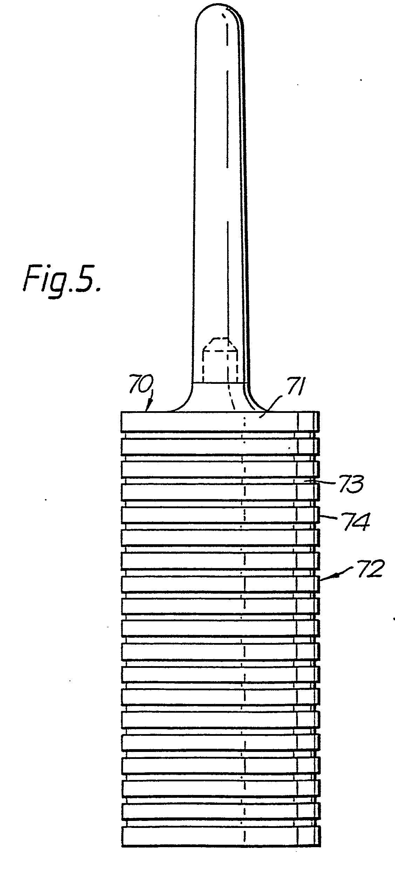

As shown in Figure 5, a needle holder 70 comprises a cap 71 and a cylindrical skirt 72. The skirt 72 is provided on its external surface with a series of grooves 73 which encircle the skirt, each lying in a plane perpendicular to the axis of the skirt. The areas of the skirt between the grooves 73 form ridges 74. The combination of ridges and grooves presents a non-slip surface on the exterior of the skirt, which can be gripped manually.

A label may be attached to the tube near to the bottom thereof so that the ribs 7 do not come into contact with it, or the glass or plastics material may be etched so that marking with a felt pen or pencil may be carried out without need for a label.

The pack contains a plurality of such blood sampling units, all ready-assembled as shown in Figure 1.

When the veterinary surgeon wants to take a blood sample, he takes an assembled unit from the pack, removes the sheath 13 (which can conveniently be broken off from the boss 17 by twisting) and holds the assembly in one hand as shown in Figure 3 with the bottom of the tube resting across the palm of the hand and the cannula resting on the extended index finger which is used to palpate a distended vein. Then the needle point 11 is introduced into the vein by moving forward the hand and assembly. Venopuncture is accomplished. Then by pressure of the palm on the base of the tube in opposition to thumb and forefinger pressure on top of the holder, the surgeon presses the tube and holder together, as shown in Figure 4. The tube slides inside the holder, air being able to escape from the interior of the holder by means of the gaps between the ribs 7. The ribs 7 make a frictional contact against the exterior surface of the tube, the friction being sufficient to prevent the tube falling out under the force of gravity but not sufficient to prevent the surgeon sliding the tube relative to the holder by means of manual pressure. The stopper 2 has a smaller external diameter than the tube so that it does not interfere with the sliding movement.

The inner needle point 12 pierces the stopper 2 and blood is sucked into the tube in the conventional manner. After the sample has been taken, the surgeon withdraws the tube from the holder and replaces the tube in hole 14 in the pack. Preferably the pack also provides a second set of holes to accommodate the holder, needle and sheath after use. The surgeon proceeds to take the next sample and uses a fresh blood-sampling unit which is ready-assembled in the pack.

In one form of the embodiment of Figures 1 and 2, the holder 3 is moulded from high density polyethylene and the needle 4 is a push-fit in an aperture passing through the cap 9 of the needle holder.

The needle may be inserted from the exterior or the interior surface of the holder. The needle is held in a suitable gripping tool having e.g. the action of a pliers and is forced through the aperture, which initially has a marginally smaller diameter than the exterior diameter of the needle. The needle may cut a thin layer of plastics material from around the aperture as it is forced through. This thin layer of plastics material may be removed from the needle tip, e.g. by an air blast.

The sleeve portion 18 on the interior surface of the cap shown in Fig. 1 may be omitted. However, it is important that the cap 9 and the boss 10 above it have sufficient length (in an axial direction) to grip the needle securely. The frictional contact between needle and holder must be sufficient to retain the needle in position when the outer needle point 11 is inserted into a vein and the inner needle point 12 pierces the stopper 2.

The needle holder may be moulded of polypropylene, but it has been found with this form of the embodiment that the accuracy of the aperture diameter is more critical with polypropylene than with polyethylene.

The needle may then be secured in the cap portion of the holder by welding e.g. sonic welding or heat welding.

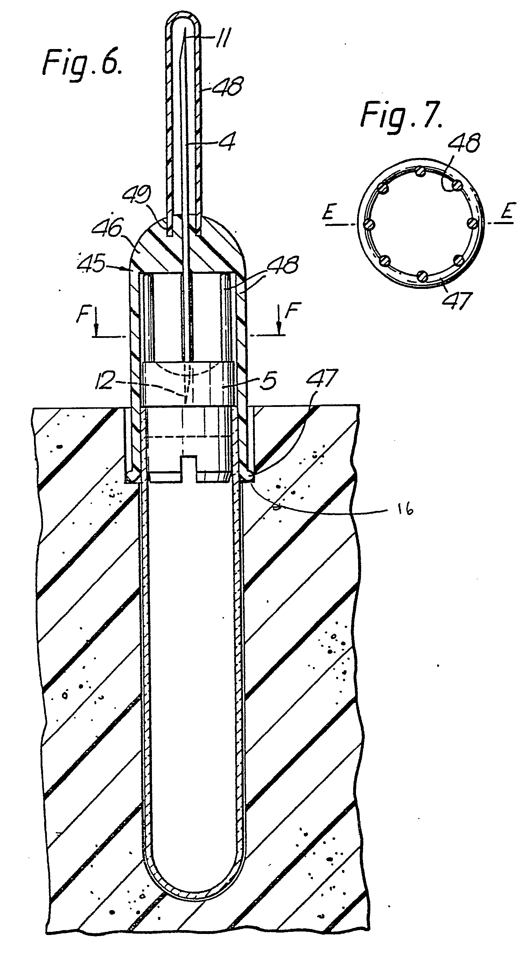

In the second embodiment, shown in Figures 6 and 7, the needle holder 45 consists of a domed cap 46 and a beaded rim 47 connected by eight longitudinal ribs 48 which form an open skirt with no plastics material between the ribs. The top surface of the cap 46 serves as a gripping surface for the surgeon's fingers. The sheath 48 for the outer end of the needle is received in an annular groove 49 in the cap. In the upper part of the hole 14 in the packaging material, the diameter of the hole is increased so that a ledge 16 is formed. The rim 47 of the holder rests on this ledge.

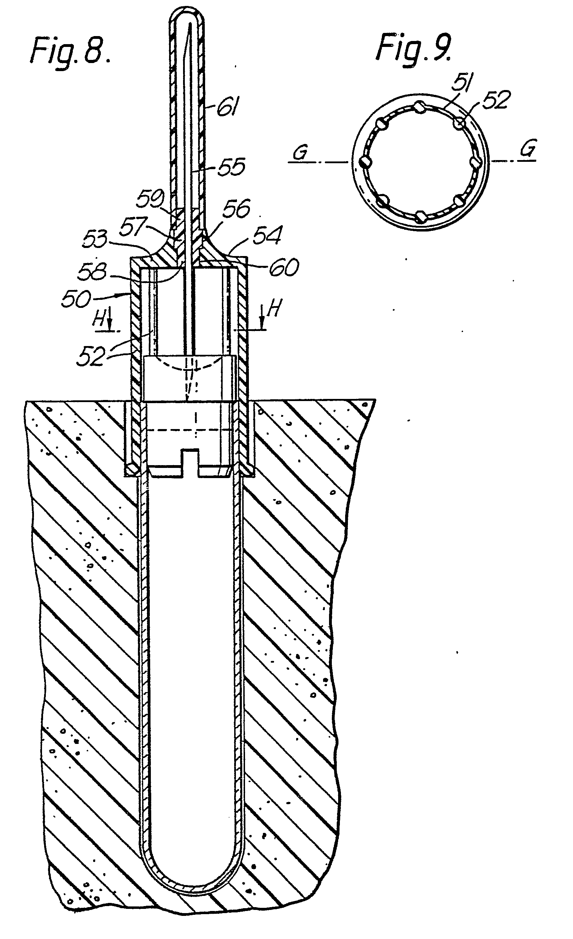

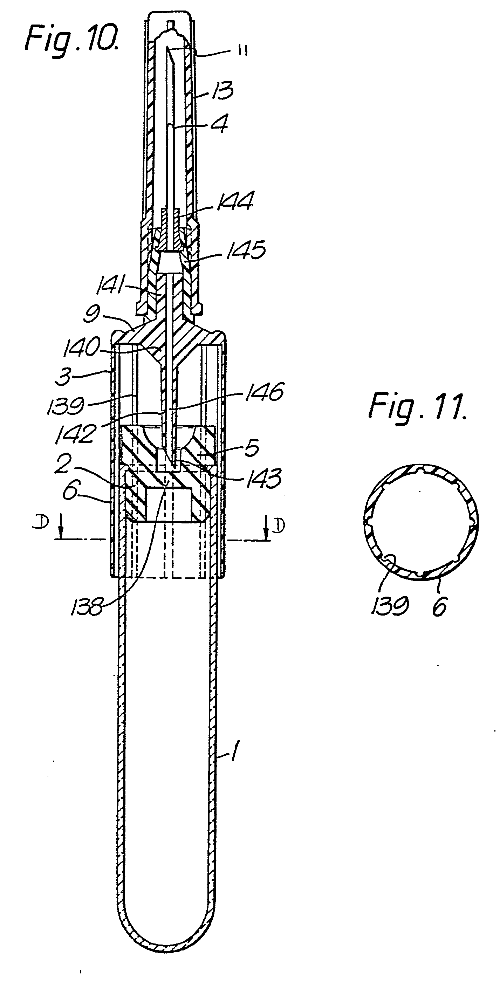

The third embodiment of the invention, shown in Figures 8 and 9, is similar to that of Figure 6 and 7 but the holder 50 has a thin walled continuous skirt 51 between the ribs 52. The cap 53 has a concave top surface 54 to provide a finger grip. The needle 55 is permanently secured in a collar 56 which has a central portion 57 of greater diameter than its two end portions 58, 59. The collar 56 is a tight fit in a bore 60 in the cap 53, the bore having an upper section of greater diameter to receive the central portion 57 and a lower section of lesser diameter to receive the lower end portion 58 so that the collar cannot be pushed downwardly into the cap 53. The sheath 61 is seated on the upper end portion 59 of the collar.

The packaging for the unit of this embodiment includes a set of recesses into which the needle holders can be placed after a sample has been taken.

The needle holders may be returned, preferably by the testing laboratory, to the manufacturer. Each used needle can then be removed from the needle holder by a tool which pulls the collar 56 upwardly out of the bore 56. A fresh sterile needle can be inserted into the needle holder which can then be assembled on to a fresh evacuated tube. If desired, the needles may be sterilized and re-used.

In the embodiment of Figures 10 and 11 the stopper 2 of elastomeric material (suitably butyl rubber) has a head portion 5. The centre of the stopper is recessed from above and below so that only a membrane 138 of elastomeric material (having a thickness of approx. 2.5 mm for example) is present on the axis of the stopper. The skirt 6 of the holder is a cylindrical shell, the internal surface of which is provided with eight longitudinal grooves 139. The lower end of the skirt is not flared or flanged.

The closed end of the holder is formed as a cap 9 having an axial cannula portion 140 moulded integrally therein. The cannula portion extends upwardly from the cap to form a boss 141 and downwardly from the cap inside the skirt 6 to form a rigid needle-like tubular projection 142 with a point end 143 cut slantwise. The outside diameter of the tubular projection 142 is approx. 2 mm. The interior surface of the skirt 6 and the exterior surface of the tubular projection 142 is suitably coated with a lubricant such as a silicone preparation which can be applied by spraying.

The needle 4 is a standard single-pointed metal needle having a metal limb 144 set into a sleeve 145 of plastics material. This sleeve seats around the boss 141 as a friction fit thereon or may be secured by adhesive, by welding or the like. The pointed end 11 of the needle is protected by a ribbed sheath 13 which fits onto the sleeve 145 and is sealed thereto during manufacture. The bore 146 passing through the cannula portion 140 of the holder communicates with the hollow interior of the needle 4 via the interior of the sleeve 145.

The blood sampling unit is supplied in a pack containing a plurality of such units, all ready-assembled as shown in Figure 1.

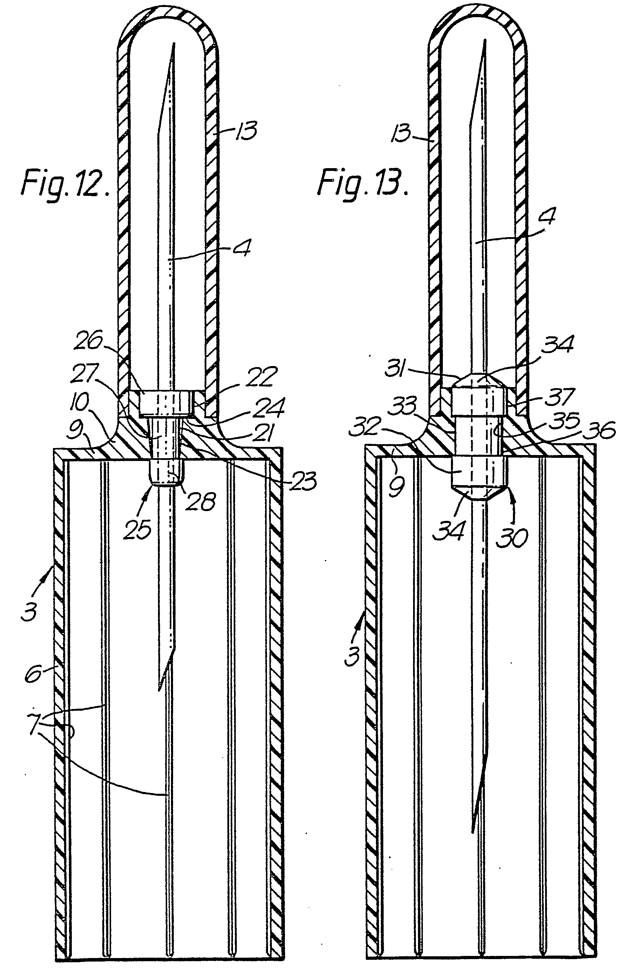

In the embodiment shown in Figure 12, the closed portion of the holder is formed as a cap 9 of solid plastics material with an axial boss 10 extending upwardly therefrom. Passing through the boss and cap there is an axial bore 21 which has an upper region 22 of larger diameter and a lower region 23 which is of smaller diameter and which, in addition, tapers slightly in the downward direction. The bottom of the upper region 22 of the bore is formed as a ledge 24.

A double ended hollow needle 4 has a rigid plastics collar 25 secured thereto, somewhat nearer the inner point 12 than the outer point 11 of the needle. The collar comprises a cylindrical body portion 26, a cylindrical neck 27 of smaller diameter and a cylindrical head 28 of greater diameter than the neck but of smaller diameter than the body portion 26. The leading edges of the body portion 26 and of the head 28 are rounded.

In manufacturing the needle holders, the collars are first secured to the needles by moulding them in position or by adhering pre-moulded collars to the needles. The needles may then be delivered to an assembly station via a sensor to determine the orientation of each needle because the needles must be presented at the assembly station with the head 28 directed towards the cap 9. The sensor may be electronic or electrical (e.g. an electric eye) or it may be a physical limit which detects needles projecting in the wrong direction and knocks them off the assembly line. At the assembly station, the collar 25 is gripped by one tool and the needle holder 3 is held in another tool while the two components are brought together. The head 28 is forced through the bore 21 until the head snaps into position below the bottom edge of the cap 9. The body portion 26 of the collar seats in the upper region 22 of the bore and prevents further downward movement of the collar because it engages ledge 24.

If desired, the collar may then be heat sealed in position.

A sheath 13 is then fitted on to the top of the boss 10 to protect the outer end 11 of the needle and heat sealed in position.

The collar may suitably be of rigid plastics material e.g. nylon or polystyrene. The holder may suitably be of polypropylene. In the case of a snap-fit engagement, the plastics material of the cap portion of the holder should be flexible enough to permit insertion of the collar.

The collar may be pre-moulded and the fixed on to the needle e.g. with adhesive. Alternatively the collar may be moulded around the needle, which suitably is formed with a means to key the collar on to the needle.

As a further alternative, the collar may be a shaped aluminium sleeve crimped on to the needle.

Figure 13 shows an alternative embodiment to that of Figure 12 in which the collar 30 has two similar heads 31, 32 separated by a neck 33 of smaller diameter. Each of the heads has a conical leading portion 34. The collar is secured in the middle of the needle 4.

The bore 35 in the cap 9 has a cylindrical lower region 36 (i.e. without a taper) and a cylindrical upper region 37 of slightly larger diameter.

In this embodiment the needle can be presented to the cap 9 in either orientation because either of the heads 3, 32 can be pushed through the bore 35 and the needle projects equally on both sides of the collar.

The use of a separate collar has been found to be more straightforward, faster and cheaper than direct moulding of the needle holder around the needle. There is less risk of the collar being dislodged than in the embodiment of Figures 8 and 9.

It is preferred to have the inner tip· of the needle embedded slightly in the stopper so that the blood path through the cannula can be kept in a sterile condition. The contact between the needle and the stopper assists in preventing the tube falling out under the force of gravity.

In any of the above embodiments, the sheath over the outer end of the needle may be replaced or supplemented by a "blister pack" of flexible plastics material sealed into position.

By use of the present invention the following advantages are achieved:

1) No time or trouble has to be spent assembling the blood sampling unit prior to use.

2) The tube is retained in the holder prior to and during insertion of the needle into a vein.

3) Used needles need not be separated from the holder because the holder is not used again, at least until after it has been returned to the factory.

4) There is no serious likelihood of a needle being used twice because each tube is equipped with a fresh needle.

5) Sliding frictional contact between the holder and the exterior of the tube over a substantial length of the holder ensures longitudinal rigidity, correct alignment and smooth movement of the tube relative to the holder.

6) The assembly can be held in and operated by one hand with fingers gripping the top of the holder and one finger can extend alongside the cannula to locate a vein. This is not possible with a standard blood sampling apparatus having a flange at the bottom of the holder because the fingers are used to grip the flange and cannot reach as far as the tip of the cannula.

Although the invention has been described with reference to use by a veterinary surgeon, the apparatus of the invention is also appropriate for use by medical personnel, verterinary inspectors and other persons who are required to take blood samples from humans or animals.

When a preferred unit according to the invention is in the preferred assembled position of Figure 1, the distance from the top surface of the cap portion 9 (excluding the boss 10 and cannula 4) to the bottom of the tube 1 may suitably be from 90 to 95 mms., particularly about 92 mms, and when the unit is in the compressed position (with the stopper flush against the underside of the cap portion) the said distance may suitably be from 82 to 87 mms., particularly about 84 mms. The holder may suitably have a length of 37 to 39 mms., particularly about 38 mms., and the tube plus stopper may suitably have an overall length from 80 to 85 mms., particularly about 82 mms.

## Claims

1. Blood sampling apparatus supplied as an assembled unit comprising an evacuated tube (1) with a stopper (2), and a needle holder (3) in which a hollow needle or cannula (4) is secured, said needle holder (3) having a skirt (6) which surrounds the stoppered end of the tube (1), characterised in that the stopper (2) has a head (5) with an external diameter not greater than the external diameter of the tube (1), and the interior of the skirt (6) of the needle holder (3) is adapted to embrace the exterior of the tube (1) but nevertheless to allow the tube (1) to slide inside the holder (3) under manual pressure.

2. Apparatus according to claim 1, wherein the interior surface of the skirt (6) has longitudinal ribs (7, 48, 52) which slidingly engage the tube (1).

3. Apparatus according to either of the preceding claims, wherein the interior of the skirt (6) embraces the exterior of the tube (1) sufficiently tightly to prevent the tube (1) falling out under the force of gravity.

4. Apparatus according to any of the preceding claims, wherein the needle holder (3) has a closed cap portion (9, 71, 46, 53), whose exterior surface is formed as a finger grip.

5. Apparatus according to claim 4, wherein the length of the holder (3) and tube (1) in the assembled unit is such that the unit can be held in one hand with fingers gripping the cap portion (9, 71, 46, 53) of the holder (3) and the palm receiving the closed end of the tube (1).

6. Apparatus according to any of the preceding claims, wherein the needle (4) is secured directly into the cap portion (9, 71, 46) of the holder (3).

7. Apparatus according to claim 6, wherein the needle (4) is secured by adhesive or welding.

8. Apparatus according· to any of claims 1—5, wherein the needle (4) is secured in a collar (56, 25, 30) which in turn is secured in a bore (60, 21, 35) in the cap portion (53, 9) of the holder (3).

9. Apparatus according to claim 8, wherein the collar (25, 30) is a snap-fit in a bore (21, 35) in the cap portion (53, 9).

10. Blood sampling apparatus in a pack comprising a plurality of assembled units according to any of the preceding claims.

## Revendications

1. Appareil de prise de sang fourni sous forme d'ensemble assemblé comportant un tube vide (1) muni d'un tampon (2), et un porte-aiguille (3) dans lequel est fixée une aiguille creuse ou canule (4), ledit porte-aiguille présentant une jupe (6) qui entoure l'extrémité bouchée du tube (1), caractérisé en ce que le tampon (2) présente une tête (5) à diamètre extérieur non supérieur au diamètre externe du tube (1), et l'intérieur de la jupe (6) du porte-aiguille (3) est agencé pour entourer l'extérieur du tube (1) mais pour laisser néanmoins le tube (1) glisser à l'intérieur du porte-aiguille (3) sous l'effet d'une pression manuelle.

2. Appareil selon la revendication 1, caractérisé en ce que la surface intérieure de la jupe (6) présente des nervures longitudinales (7, 48, 52) qui sont en contact glissant avec le tube (1).

3. Appareil selon l'une ou l'autre des revendications précédentes, caractérisé en ce que l'intérieur de la jupe (6) entoure l'extérieur du tube (1) assez étroitement pour empêcher le tube (1) de tomber à l'extérieur sous l'effet de la pesanteur.

4. Appareil selon l'une quelconque des revendications précédentes, caractérisé en ce que le porte-aiguille (3) présente une partie formant chapeau fermée (9, 71, 46, 53) dont la surface extérieure a la forme voulue pour offrir une prise aux doigts.

5. Appareil selon la revendication 4, caractérisé en ce que la longueur du porte-aiguille (3) et le tube (1) dans l'ensemble assemblé est telle que l'ensemble peut être tenu d'une main, les doigts agrippant la partie formant chapeau (9, 71, 46, 53) du porte-aiguille (3) et la paume recevant l'extrémité fermée du tube (1).

6. Appareil selon l'une quelconque des revendications précédentes, caractérisé en ce que l'aiguille (4) est directement fixée dans la partie formant chapeau (9, 71, 46) du porte-aiguille (3).

7. Appareil selon la revendication 6, caractérisé en ce que l'aiguille (4) est fixée par adhésif ou soudage.

8. Appareil selon l'une quelconque des revendications 1 à 5, caractérisé en ce que l'aiguille (4) est fixée dans un collier (56, 25, 30) lui-même fixé dans un alésage (60, 21, 35) de la partie formant chapeau (53, 9) du porte-aiguille (3).

9. Appareil selon la revendication 8, caractérisé en ce que le collier (25, 30) est emboîté élastiquement dans un alésage (21, 35) de la partie formant chapeau (53, 9).

10. Appareil de prise de sang incorporé à un paquet comprenant une pluralité d'ensembles assemblés selon l'une quelconque des revendications précédentes.

**Patentansprüche**

1. Vorrichtung zur Entnahme von Blutproben in Form einer (zusammengesetzten) Einheit, umfassend ein evakuiertes Röhrchen (1) mit einem Stopfen (2) und einem Nadelhalter (3), in dem eine Hohlnadel oder Kanüle (4) sicher befestigt ist, wobei der Nadelhalter (3) eine Hülse (Rand) (6) aufweist, der das mit dem Stopfen verschlossene Ende des Röhrchens (1) umgibt, dadurch gekennzeichnet, daß der Stopfen (2) ein Kopfstück (5) umfaßt mit einem äußeren Durchmesser, der nicht größer ist also der äußere Durchmesser des Röhrchens (1) und wobei das Innere der Hülse (6) des Nadelhalters (3) das Äußere des Röhrchens (1) umfassen kann, wobei jedoch das Röhrchens (1) unter manuellem Druck in den Halter (3) leiten kann.

2. Vorrichtung nach Anspruch 1, wobei die Innenseite der Hülse (6) längsverlaufende Rippen (7, 48, 52) aufweist, die verschiebbar in das Röhrchen (1) umschließen.

3. Vorrichtung nach einem der vorangehenden Ansprüche, wobei das Innere der Hülse (6) das Äußere des Röhrchens (1) ausreichend fest umschließt, um ein Herausfallen unter der Schwerkraft zu vermeiden.

4. Vorrichtung nach einem der vorangehenden Ansprüche, wobei der Nadelhalter (3) einengeschlossenen Kappenteil (9, 71, 46, 53) umfaßt, dessen Außenseite also Griff für den Finger ausgebildet ist.

5. Vorrichtung nach Anspruch 4, wobei die Länge des Halters (3) und des Röhrchens (1) in der zusammengesetzten Einheit so ist, daß die Einheit, so mit einer Hand gehalten werden kann, daß die Finger den Kappenteil (9, 71, 46, 53) des Halters (3) greifen und die Handfläche das geschlossene Ende des Röhrchens (1) aufnimmt.

6. Vorrichtung nach einem der vorangehenden Ansprüche, wobei die Nadel (4) direkt in dem Kappenteil (9, 71, 46) des Halters (3) befestigt ist.

7. Vorrichtung nach Anspruch 6, wobei die Nadel (4) durch Ankleben oder Anschweißen sicher befestigt ist.

8. Vorrichtung nach einem der Ansprüche 1 bis 5, wobei die Nadel (4) in einer Manschette (56, 25, 30) sicher befestigt ist, die ihrerseits in einer Öffnung (60, 21, 35) in dem Kappenteil (53, 9) des Halters (3) sicher befestigt ist.

9. Vorrichtung nach Anspruch 8, wobei die Manschette (25, 30) in eine Bohrung (21, 35) in dem Kappenteil (53, 9) einschnappt.

10. Vorrichtung zur Blutentnahme in einer Packung, umfassend eine Vielzahl zusammengesetzter Einheiten nach einem der vorangehenden Ansprüche.

Fig.1.

Fig.2.

Fig. 3.

Fig. 4.

2

Fig.5.

Fig. 6.

Fig. 7.

Fig. 8.

Fig. 9.

61

55

59

53  57

56

54

58

50

60

52

H

H

51

52

G

G

Fig. 10.

Fig. 11.

6

0 026 679

Fig.12.

Fig.13.

7